(19) Europäisches Patentamt
European Patent Office
Office européen des brevets

(11) EP 0 579 806 B1

(12) EUROPÄISCHE PATENTSCHRIFT

(45) Veröffentlichungstag und Bekanntmachung des Hinweises auf die Patenterteilung:
**07.07.1999 Patentblatt 1999/27**

(21) Anmeldenummer: **93903168.8**

(22) Anmeldetag: **12.02.1993**

(51) Int. Cl.[6]: **A61K 31/505**, A61K 31/70, A61K 31/68

(86) Internationale Anmeldenummer:
**PCT/DE93/00123**

(87) Internationale Veröffentlichungsnummer:
**WO 93/15738 (19.08.1993 Gazette 1993/20)**

# (54) ARZNEIMITTEL ZUR BEHANDLUNG UND/ODER PROPHYLAXE VON STÖRUNGEN DES FOLATSTOFFWECHSELS

MEDICAMENT FOR THE TREATMENT AND/OR PROPHYLAXIS OF FOLATE METABOLISM DISORDERS

MEDICAMENT POUR LE TRAITEMENT ET/OU LA PROPHYLAXIE DES TROUBLES DU METABOLISME DU FOLATE

(84) Benannte Vertragsstaaten:
**AT BE CH DE DK ES FR GB GR IE IT LI LU MC NL PT SE**

(30) Priorität: **12.02.1992 DE 4204108**

(43) Veröffentlichungstag der Anmeldung:
**26.01.1994 Patentblatt 1994/04**

(73) Patentinhaber: **Kragler, Peter**
**80333 München (DE)**

(72) Erfinder:
- **Waldthaler, Urban, Dr.**
  **86159 Augsburg (DE)**
- **Dierig, Klaus-Uwe**
  **86391 Stadtbergen (DE)**

(74) Vertreter:
**Kraus, Walter, Dr. et al**
**Patentanwälte Kraus, Weisert & Partner**
**Thomas-Wimmer-Ring 15**
**80539 München (DE)**

(56) Entgegenhaltungen:
**EP-A- 0 037 488**

- **DATABASE WPIL Week 8610, Derwent Publications Ltd., London, GB; AN 86-065707**
- **BIOL. CHEM. HOPPE-SEYLER Bd. 369, Nr. 3, 1988, Seiten 143 - 148 W. DR\GE ET AL 'ABNORMAL AMINO-ACID CONCENTRATIONS IN THE BLOOD OF PATIENTS WITH ACQUIRED IMMUNODEFICIENCY SYNDROME (AIDS) MAY CONTRIBUTE TO THE IMMUNOLOGICAL DEFECT'**
- **AM. J. HEMATOL. Bd. 33, Nr. 3, 1990, Seiten 177 - 183 B. C. HERZLICH ET AL. 'SYNERGY OF INHIBITION OF DNA SYNTHESIS IN HUMAN BONE MARROW BY AZIDOTHYMIDINE PLUS DEFICIENCY OF FOLATE AND/OR VITAMIN B12?'**
- **ARCH. NEUROL. Bd. 49, Nr. 6, 1992, Seiten 649 - 652 E.H. REYNOLDS ET AL. 'VITAMIN B12 METABOLISM IN MULTIPLE SCLEROSIS'**
- **DIALOG INFORMATION SERVICES, FILE 155 MEDLINE DIALOG ACCESSION NO. 05483795**
- **LANCET Bd. 2, Nr. 8557, 1987, Seite 509 J.A. BLAIR 'FOLATE DEFICIENCY AND DEMYELINATION IN AIDS' in der Anmeldung erw{hnt**
- **NUTR. CANCER Bd. 3, Nr. 4, 1982, Seiten 216 - 222 D. M. DISORBO ET AL. 'VITAMIN B6 KILLS HEPATOMA CELLS IN CULTURE'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

- **MEDICAL HYPOTHESES Bd. 38, Nr. 1, Mai 1992, Seiten 11 - 19 B. REGLAND ET AL. 'SLOWED SYNTHESIS OF DNA AND METHIONINE IS A PATHOGENETIC MECHANISM COMMON TO DEMENTIA IN DOWN'S SYNDROME, AIDS AND ALZHEIMER'S DISEASE?'**
- **JAMA Bd. 259, Nr. 10, 1988, Seiten 1525 - 1530 D.C. HEIMBURGER ET AL. 'IMPROVEMENT IN BRONCHIAL SQUAMOUS METAPLASIA IN SMOKERS TREATED WITH FOLATE AND VITAMIN B12'**

Bemerkungen:

Die Akte enthält technische Angaben, die nach dem Eingang der Anmeldung eingereicht wurden und die nicht in dieser Patentschrift enthalten sind.

## Beschreibung

Die Erfindung betrifft die Verwendung bestimmter Verbindungen zur Herstellung eines Arzneimittels zur Behandlung und/oder Prophylaxe Von Störungen des Folat-Stoffwechsels, insbesondere zur Behandlung und/oder Prophylaxe von AIDS oder Aids-Related-Complex (ARC), Syphilis, Chronic-Immuno-Deficiency-Syndrome (CIDS) bzw. Chronic-Fatigue-Syndrome (CFS), multipler Sklerose (MS), Neoplasien und Malaria.

Die Folsäure und ihre Derivate sind in der Natur verbreitet und für den Menschen ein essentieller Nahrungsfaktor. Die Folsäure der folgenden Formel I selbst ist ein Vitamin, dessen Mangel bei Mensch und Tier Wachstumsschwäche und verschiedene Formen der Anämie hervorruft:

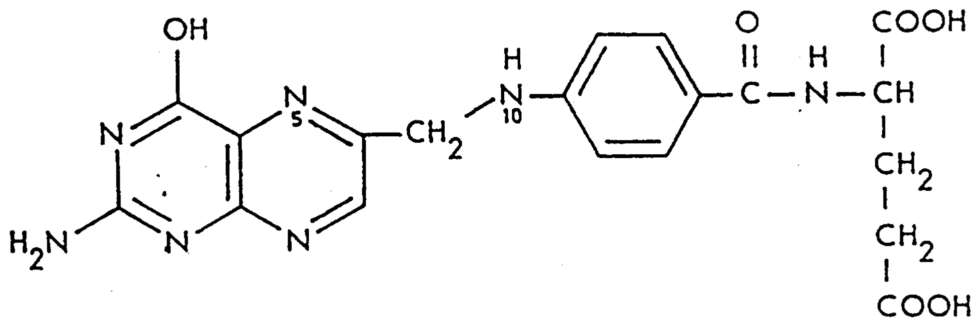

Pteridinrest

p-Amino-benzoesäurerest

Glutaminsäurerest

Folsäure
(Pteroylglutaminsäure)

Die Bezeichnung Folsäure wird für alle Verbindungen verwendet, die als chemischen Bestandteil einen Pteridinrest, einen para-Aminobenzoesäurerest und einen oder mehrere Glutaminsäurereste enthalten. In höheren Organismen herrscht jedoch das Monoglutamat vor. Die Folsäure nimmt im Stoffwechsel der Ein-Kohlenstoffeinheiten eine zentrale Position ein. Sie ist Coenzym von Methyl-, Methenyl-, Methylen- und Formylgruppen übertragenden Enzymen und somit an zahlreichen Biosyntheseprozessen, insbesondere des Amonisäure- und Nukleotid-Stoffwechsels beteiligt.

Die biologisch wirksame Form der Folsäure sind die 5,6,7,8-Tetrahydroderivate (5,6,7,8-Tetrahydro-pteroylglutamat bzw. 5,6,7,8-Tetrahydro-pteroyl-poly-gamma-glutamat, Tetrahyrdofolsäure). Insbesondere ist die Folsäure an der Synthese biologischer Makromoleküle im Körper, wie von Proteinen, DNA und RNA, beteiligt. Die beigefügte Figur 1 zeigt eine Übersicht über den Teil des Folsäure-Stoffwechsels, der mit der Makromolekularsynthese assoziiert ist. Wie daraus hervorgeht, nehmen die Enzyme Homocystein-Methioninmethyltransferase (EC 2.1.1.13) und Thymidylatsynthetase (EC 2.1.1.45) eine zentrale Rolle ein.

Die Thymidylatsynthetase führt dabei zu den Nukleotiden der Thymidinreihe. Diese Reaktionen werden als obligatorische DNA-Methylierung bezeichnet. Es handelt sich dabei um eine Methylierung von 2,4-Di-hydroxy-pyrimidin in Position 5 mit der Zielverbindung 5-Methyluracil = Thymin. Die Homocystein-Methioninmethyltransferase führt zum Stoffwechselweg der sogenannten DNA-Methylierung, d.h. der Methylierung von 2-Hydroxy-4-amino-pyrimidin in Position 5 mit der Zielverbindung 5-Methylcytosin. Dabei dient Vitamin $B_{12}$ als Cofaktor der Homocystein-Methioninmethyltransferase.

Methionin ist dabei an Reaktionen beteiligt, die der Regeneration des Folat-Coenzyms (der 5-Methyl-tetrahydropteroyl-glutaminsäure) dienen.

Die Funktion einer Zelle ist sowohl von der obligatorischen, als auch von der fakultativen Methylierung abhängig. Dadurch entsteht das spezielle DNA-Methylierungsmuster, wodurch die Transkription und die Translation, d.h. die Genexpression, reguliert werden.

Der tägliche Bedarf an Folsäure liegt beim Menschen bei 25 bis 250 μg pro Tag. Er kann durch Verabreichung verschiedener Medikamente, wie Tuberculostatika oder Contraceptiva, erhöht werden.

Vitamin $B_6$ oder Pyridoxin, das im Körper in die Wirkform Pyridoxalphosphat (PLP) überführt wird, ist ein Coenzym des Aminosäure-Stoffwechsels. In dem in Figur 1 betrachteten Fall wirkt Vitamin $B_6$ als Coenzym der Serinhydroxymethyltransferase.

Erkrankungen wie AIDS, ARC (Aids-Related-Complex), Syphilis, CIDS (Chronic-Immuno-Deficiency-Syndrome), CFS (Chronic-Fatigue-Syndrome), multiple Sklerose, Krebs und Malaria treten in jüngerer Zeit verstärkt auf. Bis heute konnte für derartige Erkrankungen keine zufriedenstellende Therapie entwickelt werden.

So können AIDS, ARC und CIDS bisher nur symptomal und in unzureichendem Maß behandelt werden. Dies geschieht beispielsweise durch die Verabreichung falscher Nukleotide, z.B. Azidothymidin. Eine Heilung derartiger Erkrankungen ist bisher noch nicht beobachtet worden.

Krebs kann heute zum Teil erfolgreich strahlentherapeutisch oder chemotherapeutisch, d.h. mit Cytostatika, behandelt werden. Derartige Behandlungsmethoden sind jedoch oft langwierig und für den Patienten mit erheblichen Beeinträchtigungen und Nebenwirkungen verbunden. So ist zum Beispiel die Strahlentherapie mit erheblicher mutagener, karzinogener oder teratogener Wirkung verbunden. Bei der Chemotherapie werden zum Teil erhebliche psychische und körperliche Beeinträchtigungen des Allgemeinbefindens des Patienten beobachtet. So kann es beispielsweise zu Anämien, gastrointestinalen Störungen, einer Beeinträchtigung der Gonadenfunktion, zu Haarausfall, zu Nierenversagen und zu Organschäden an verschiedenen inneren Organen, wie an Herz, Lunge, Leber und dem Nervensystem, kommen.

Häufig wird auch eine Resistenz der Krebszellen gegenüber Cytostatika beobachtet, was die weitere Therapie erheblich erschwert. So ist zum Beispiel eine erworbene Resistenz von Tumorzellen gegenüber Folat-Antagonisten aus der Druckschrift Folate Antagonists as Therapeutic Agents, Band 1, Seite 317 ff. bekannt. Diese Resistenz wird auf eine Amplifikation des Dihydrofolat-Reduktase-Gens zurückgeführt.

Bei der Syphilis handelt es sich um eine durch Treponema pallidum hervorgerufene Geschlechtskrankheit. Bisher wurde sie nur unzureichend mit β-Lactam-Antibiotika, wie Penicillin und Cephalosporin, behandelt. Wie aus einer Zellkultur mit durch Treponema pallidum infizierten Kaninchenzellen hervorgeht, sind die Krankheitssymptome der Syphilis vermutlich auf eine Hemmung der Makromolekularsynthese zurückzuführen (Infection and Immunity, August 1983, Band 41, Nr. 2, Seiten 636 bis 643). Als Ursache dieser Wirkung vermutet man ein vom Erreger gebildetes Toxin.

Bei dem Chronic-Fatigue-Syndrome (CFS), einem durch abnorme Müdigkeit und Lethargie gekennzeichneten Zustand, handelt es sich vermutlich um eine Erkrankung des Immunsystems, bei der im Blut des Patienten Genfragmente, die sich von dem HTLV-2-Virus ableiten, gefunden wurden (Newsweek, 12. November 1990, Seiten 62 bis 70).

Bei der Malaria handelt es sich um eine Infektionskrankheit, die durch Protozoen der Gattung Plasmodium, die durch Anopheles-Mücken übertragen werden, hervorgerufen wird. Trotz intensiver Bekämpfungsmaßnahmen ist die Malaria heute weltweit in den Tropen und zum Teil auch in den Subtropen verbreitet. Malariaprophylaxe und -therapie wird durch Arzneimittel, wie Chloroquin, Primaquin, Proguanil und Pyrimethamin, betrieben. Trotzdem sterben jährlich weltweit über eine Million Menschen an der Malaria.

Hinweise auf einen Zusammenhang zwischen dem Folat-Stoffwechsel und Krebs bzw. AIDS sind aus dem Stand der Technik nur unzureichend zu entnehmen. So wird eine Verbesserung der Metaplasie (Gewebsneubildung) eines normalerweise mit Zylinderepithel ausgekleideten Lungenbronchus bei Rauchern durch Gabe von Folat und Vitamin $B_{12}$ im JAMA, 11. März 1988, Band 259, Nr. 10, Seiten 1525 bis 1530, beschrieben.

Auf einen Zusammenhang zwischen AIDS und Folatmangel wird in The Lancet, 29. August 1987, Seite 509, hingewiesen.

Der vorliegenden Erfindung liegt die Aufgabe zugrunde, ein Arzneimittel und die Verwendung bestimmter Verbindungen zur Behandlung und/oder Prophylaxe für die obengenannten Krankheiten zur Verfügung zu stellen.

Das erfindungsgemäße Arzneimittel soll für den Patienten mit keinen psychischen und physischen Belastungen verbunden sein. Das Arzneimittel soll in einfacher Weise, beispielsweise in Form von Tabletten, verabreicht werden, Soweit wie möglich soll bei dem erfindungsgemäßen Arzneimittel und bei der erfindungemäßen Verwendung eine stationäre Behandlung vermieden werden.

Nebenwirkungen, die bei den bekannten Arzneimitteln beispielsweise bei der Behandlung von Krebs auftreten, wie Haarausfall oder Erbrechen, sollten bei dem erfindungsgemäßen Arzneimittel bzw. bei der erfindungsgemäßen Verwendung vermieden werden.

In dem erfindungsgemäßen Arzneimittel sollen Stoffe eingesetzt werden, die den Körper nicht belasten und vorzugsweise körpereigene Stoffe sind oder Stoffe sind, die schon seit langem als Arzneimittel für andere Indikationen, beispielsweise als Vitamine, verwendet werden.

Überraschenderweise wurde gefunden, daß Erkrankungen wie AIDS und Aids-Related-Complex (ARC), Syphilis, Chronic-Immuno-Deficiency-Syndrome (CIDS) bzw. Chronic-Fatigue-Syndrome (CFS), multiple Sklerose (MS), Neoplasien und Malaria mit einer Störung des Folat-Stoffwechsels assoziiert sind und erfolgreich behandelt werden können, wenn Verbindungen eingesetzt werden, die in den Folat-Stoffwechsel eingreifen.

Gegenstand der Erfindung ist die Herstellung eines Arzneimittels, das dadurch gekennzeichnet ist, daß es mindestens eine Verbindung aus der Gruppe Thymin, Thymidin, desoxy-Thymidin, desoxy-Thymidin-mono-, -di-, triphosphat und ein pharmazeutisch annehmbares Salz davon sowie übliche Träger und/oder Verdünnungsmittel enthält.

Es ist bevorzugt, daß das erfindungsgemäße Arzneimittel zusätzlich mindestens eine Verbindung aus der Gruppe Methionin, Vitamin $B_{12}$, Vitamin $B_6$ und eines seiner pharmazeutisch annehmbaren Salze enthält.

Es ist weiterhin bevorzugt, daß das erfindugnsgemäße Arzneimittel, welches mindestens eine Verbindung aus der Gruppe Thymin, Thymidin, desoxy-Thymidin, desoxy-Thymidinmono-, -di-, -triphosphat und ein pharmazeutisch annehmbares Salz davon sowie übliche Träger und/oder Verdünnungsmittel enthält, oder daß das Arzneimittel, welches die genannten Verbindung und zusätzlich mindestens eine Verbindung aus der Gruppe Methionin, Vitamin $B_{12}$, Vitamin $B_6$ und ein pharmazeutisch annehmbares Salz davon enthält, zusätzlich Penicillin G und ein pharmazeutisch annehmbares Salz davon enthält.

Gegenstand der Erfindung ist die Verwendung von mindestens einer Verbindung aus der Gruppe

A. Vitamin $B_6$, Vitamin $B_{12}$, Methionin und eines pharmazeutisch annehmbaren Salzes davon
zusammen mit mindestens einer Verbindung aus der Gruppe

B. Thymin, Thymidin, desoxy-Thymidin, desoxy-Thymidin-mono-, -di- und -triphosphat und eines pharmazeutisch annehmbaren Salzes davon
und gegebenenfalls

C. mit Penicillin G und/oder einem pharmazeutisch annehmbaren Salz davon
zur Herstellung eines Arzneimittels
zur Behandlung und/oder Prophylaxe von Störungen des Folat-Stoffwechsels, wobei die Verwendung der Verbindungen aus der Gruppe A. und B. gleichzeitig, getrennt oder zeitlich abgestuft erfolgen kann und die Verwendung von Penicillin G zusammen mit der Verwendung der Verbindungen aus der Gruppe A. und/oder aus der Gruppe B. erfolgen kann.

Mit den erfindungsgemäßen Arzneimitteln und gemäß der erfindungsgemäßen Verwendung können Erkrankungen wie AIDS, ARC, CIDS, CFS, Krebs, Malaria oder Syphilis prophylaktisch oder therapeutisch behandelt werden. Bei den erfindungsgemäßen Arzneimitteln und bei der erfindungsgemäßen Verwendung handelt es sich um körpereigene Stoffe oder um Stoffe, die schon seit langer Zeit bei der Behandlung von Menschen verwendet werden. Der Metabolismus dieser Stoffe ist somit gut bekannt und führt zu keinerlei Nebenwirkungen.

Im Gegensatz zu den bisherigen Behandlungsformen dieser Erkrankungen sind die Verabreichung des erfindungsgemäßen Arzneimittels und die erfindungsgemäße Verwendung nicht mit den üblichen psychischen und physischen Belastungen für den Patienten verbunden. Das Arzneimittel kann in einfacher Weise z.B. in Form einer Tablette, verabreicht werden; eine stationäre Behandlung ist in den meisten Fällen nicht erforderlich. Da es sich um körpereigene Stoffe handelt. ist die Einnahme des erfindungsgemäßen Arzneimittels nicht mit unangenehmen Nebenwirkungen, wie Haarausfall bei der Chemotherapie, verbunden.

Gemäß einer bevorzugten Ausführungsform betrifft die vorliegende Erfindung die Verwendung der Verbindung der Gruppen A., B. und C. zur Behandlung und/oder Prophylaxe von AIDS und Aids-Related-Complex (ARC), Syphilis, Chronic-Immuno-Deficiency-Syndrome (CIDS) bzw. Chronic-Fatigue-Syndrome (CFS), multipler Sklerose (MS), Neoplasien und Malaria, wobei die Verwendung bei Neoplasien und Malaria ohne die Verwendung der Verbindungen aus der Gruppe C. erfolgt.

Die Anmelder haben die Basaltransformation von Lymphozyten von AIDS Patienten und deren Stimulierbarkeit durch Lektine an Hand der quantitativen Inkorporation von radioaktiv marklertem Thymidin in die DNA der Lymphozyten gemessen.

Dabei wurde überraschenderweise gefunden, daß unstimulierte Lymphozyten bis 300% mehr Thymidin inkorporierten als die gesunder Patienten. Dies legt einen Thymidinmangel nahe. Regt man dagegen derartige Lymphozyten mit Lektinen zur Proliferation an und gibt radioaktiv markiertes Thymidin zu, so wird nur eine schwache Stimulierbarkeit und kein Thymidineinbau beobachtet.

Wie bereits ausgeführt, kann die Ursache der Störung des Folat-Stoffwechsels entweder im Bereich der fakultativen oder im Bereich der obligatorischen DNA-Methylierung liegen (vgl. Figur 1: die rechte Seite entspricht dem Bereich der fakultativen DNA-Methylierung, die linke Seite entspricht dem Bereich der obligatorischen DNA-Methylierung).

Folgende Laborparameter wurden vor und nach der Therapie an 23 HIV-positiven Patienten mit verschiedener klinischer Symptomatik bestimmt:

1. Rotes Blutbild und Megakaryozytenfunktion
2. Weißes Blutbild und Lymphozytensubpopulation
3. Lymphozytenstimulation
4. Proteine

   a) Gesamteiweiß

b) Elektrophorese
c) Immunelektrophorese
d) zirkulierende Immunkomplexe (CIC)

5. $B_{12}$-Gehalt

a) Serum pg/ml
b) Erythrozyten pg/ml Zellen
c) mononukleäre Zellen pg/ml Zellen

6. Folat-Gehalt

a) Serum ng/ml
b) Erythrozyten ng/ml Zellen
c) mononukleäre Zellen ng/ml Zellen

7. HIV (Elisa) in Titerstufen
8. HIV - Westernblot (P24-core, P55-gag, gP41-env, gP120-env, gP160-env) mit Bewertung der Banden von + bis ++++
9. $\beta_2$-Mikroglobulin korreliert

a) mit der Aktivität der NK-Zellen (ADCC = antibody dependent cellmediated cytotoxicity)
b) mit der Aktivität der $CD_8$- NK-Zellen

10. Neopterin
Zwischenprodukt der Reaktion GPT (Guanosintriphosphat)
--> Tetrahydrobiopterin (Hydroxylierung von Phenylalanin, Tyrosin und Tryptophan)
Die Bedeutung von Neopterin ist zur Zeit noch Gegenstand wissenschaftlicher Untersuchungen.
11. Lysozym (Muramidase)
Messung der Granulozytenaktivität
12. Serologie sexuell übertragbarer Krankheiten (STD)

Zu den Laborparametern ist im einzelnen folgendes auszuführen:

zu 1.: Die Produktion von Erythrozyten (2 Millionen/sek.) im Knochenmark ist die größte Zellneubildung des menschlichen Organismus. Bei den meisten der therapierten Patienten fand sich eine leichte makrozytäre, hyperchrome Anämie bedingt durch nucleo-cytoplasmäre Asynchronität (DNA-Synthese↓, Proteinsynthese <->). Dies führt zu einer Vergrößerung des mittleren Erythrozytenvolumens (MCV↑) und einer Erhöhung des Hämoglobingehaltes des Einzelerythrozyten ($Hb_e$`↑).
Bei mit einem chain-ternimator (z.B. AZT) vorbehandelten Patienten waren diese Befunde noch ausgeprägter.
Kaschiert wurde diese Anämie häufig durch Eisenmangel, Störungen der Eisenspeicherung (Ferritin) und des Eisentransports (Transferrin).
Bei allen Patienten war nach Therapie das rote Blutbild gebessert bzw. normalisiert. Die polyploiden Megakaryozyten sind die einzigen blutbildenden Zellen, die das Knochenmark nicht verlassen.
Die bei einigen Patienten zu beobachtenden Störungen der Thrombozytensequestration -> PDW (Platelet diameter width = Streuung der Thrombozytengröße↑), sowie der Thrombozytopoese (Thrombo.< 140 00 µl) war das größte therapeutische Problem.
Besserungen traten immer ein, vollständige Normalisierungen waren selten.
Ursache: Störungen bei der endomitotischen Bildung des diplo- bzw. tetraploiden Chromosomensatzes im Megakarypblasten.
Die Analyse dieses Laborparameters läßt eine Störung im Bereich der obligatorischen DNA-Methylierung vermuten.

zu 2.: Die gravierendsten Störungen im weißen Blutbild fanden sich in der Lymphozytensubpopulation, speziell bei den $CD_4$-Zellen (sog. Helferzellen). Abgesehen davon, daß ihre absolute Zahl

im unteren oder unterhalb des Normbereiches lag, war auch ihre Funktionsfähigkeit beeinträchtigt.

Die Aufgabe der $CD_4$-Zellen liegt in der Informationsübertragung bei der Spezifizierung sowohl der humoralen als auch der zellvermittelten Immunantwort.

Auf den $CD_4$-Zellen finden sich verschiedene Rezeptoren, z.B. für IgM, für die Histokompatibilitätsantigene, für die B-Zellen oder für die $CD_8$-Natural-Killer-Zellen (= $CD_8$-cytotoxische Zellen).

Nach dem derzeitigen Wissensstand sind die polymorphkernigen neutrophilen Granulozyten die ersten Zellen mit Antigenkontakt, die Makrophagen die Zellen, die für das Processing des Antigens verantwortlich sind.

Bei allen Patienten lag vor der Therapie die Anzahl der funktionstüchtigen $CD_4$-Zellen (= B=inducer-Rezeptor$^+$, $CD_8$-inducer-Rezeptor$^+$) weit unter 50%, nach Therapie weit über 50%, meist bei 100%.

Ein derartiger Befund läßt eine Störung im Bereich der obligatorischen DNA-Methylierung erkennen.

Die $CD_8$-Suppressorzellen besitzen Rezeptoren für die Fc-Stücke von Immunglobulienen der Klasse G. Durch diese werden sie aktiviert, d.h. die $CD_8$-Suppressorzellen exprimieren den Haupt-Histokompatibilitätsantigenkomplex II. Wie die immunsuppressive Wirkung zustande kommt, ist zur Zeit noch nicht sicher bekannt. Die hohe Anzahl aktiviercer $CD_8$-Suppressorzellen korrelierte mit der polyklonalen Gammopathie der Patienten. Ein Absinken dieser überhöhten Fraktion der Lymphozytensubpopulation und damit auch eine Verbesserung der sogenannten T-Helfer/Suppressor-Ratio nach Therapie erfolgte sehr langsam, über, Monate. Einige Normalisierungen ($CD_8$-Zellen absolut < 1360/µl) nach der Therapie fanden sich erst nach einem Jahr.

Diese Befunde lassen auf eine Störung sowohl der fakultativen als auch der obligatorischen DNA-Methylierung schließen.

zu 3.: Wie bereits beschrieben, wird die Basaltransformation von Lymphozyten und deren Stimulierbarkeit durch Lektine an Hand der quantitativen Inkorporation von radioaktiv marklertem Thymidin ($H^3$-Thymidin) in die DNA der Lymphozyten gemessen. Die Maßeinheit sind Counts per Minute (cmp).

Die Stimulation

mit PHA (Phytohämagglutinin) korreliert mit der Stimulation von T-Zellen (vorwiegend $CD_4$)
mit Con A (Concanavalin A) von T-Zellen (vorwiegend $CD_8$)
mit PMW (pokeweed mitogen) von B-Zellen (T-abhängig)

Grundsätzlich geben diese Messungen Aufschluß über Störungen des de novo pathway bei der DNA-Synthese.

Basaltransformation:

Außer bei den wenigen Patienten mit ausgeprägter Lymphozytose war die Inkorporation von $H^3$-Thymidin vor der Therapie auf das 2- bis 3fache der Norm erhöht.

Lektinstimulation:

Vor Therapie bei allen Patienten desolat: Mit einigen Ausnahmen von PHA war die Stimulierbarkeit der Lymphozyten meist unterhalb 10% der Norm.

Diese Befunde sprechen für eine Störung im Bereich der obligatorischen DNA-Methylierung.

Die Normalisierung von Basaltransformation und Lymphozytenstimulation nach Therapie erfolgt (wie bei dem allmählichen Zurückgehen der $CD_8$-S-Zellen) oft erst nach Monaten bzw. nach einem Jahr.

zu 4.: Bei allen behandelten Patienten fand sich eine Erhöhung des Gesamteiweißes im Serum.

Die Analyse der Dysproteinämie in der Elektrophorese (prozentuale Verteilung der Serumproteine) zeigt, daß die Erhöhung des Gesamteiweißes durch die Überproportionierung der Gamma-Globulin-Fraktion bedingt war.

Die Analyse der Immunelektrophorese zeigte, daß IgA, IgM, im wesentlichen aber IgG weit über die Norm erhöht waren.

Nach Information durch $CD_4$-B-inducer-Zellen klonieren B-Zellen zunächst (DNA-Synthese), dann werden sie zu Antikörper produzierenden Plasmazellen (Proteinsynthese).

Die zur Vereinfachung der Schematik ausgeklammerten, zur Spezifizierung der Immunantwort

aber notwendigen Monozyten/Makrophagen sind Zellen, die zunächst Proteine synthetisteren, u.a. z.B. Interleukin 1, in aktiviertem Zustand aber auch DNA.
Störungen bei der Produktion spezifischer Antikörper lassen sich dafür, wie folgt, charakterisieren:

1. Proteinsynthese Makrophagen↓
z.B. kein Interleukin 1
2. DNA-Synthese Makrophage↓
kein Antigenprocessing
3. Zell zu Zellkontakt Makrophage-$CD_4$-B-inducer-Zelle:

   a) Antigenspezifizierung↓
   b) Rezeptorausbildung $CD_4$-Zelle↓

4. Zell zu Zellkontakt $CD_4$-B-inducer-Zelle-B-Zelle:

   a) Antigenspezifizierung↓
   b) Rezeptoren↓

5. Klonierung der B-Zelle↓ --> Inkorporation von dUTP statt dTTP --> Kettenabriß
6. Produktion von unspezifischen Antikörpern

Zirkurierende Immunkomplexe (CIC) weisen auf eine Dysproportion zwischen Antigen und spezifischen Antikörpern hin. Die Zirkulation dieser AG-AK-Komplexe ist bedingt durch eine reduzierte Präsenz der Fc-Stücke, die den Anreiz zur Phagozytose dieser Komplexe bilden.
Nach Therapie stiegen die CIC's meist an, fielen dann aber nach Monaten ab bei manchen Patienten bis in den Normbereich.
Bei späterem Wiederansteigen der zirkulierenden Immunkomplexe enthüllte die verfeinerte Fremdanamnese kein medizinisches, sondern ein gesellschaftliches Phänomen: Jeder der therapierten Patienten hatte früher oder später seine promiskuitive Lebensweise wieder aufgenommen.
Diese Befunde lassen eine Störung sowohl im Bereich der fakultativen als auch der obligatorischen DNA-Methylierung erkennten.

zu 5. und 6.: Ende 1986 fielen bei der Analyse der Labordaten prefinaler Aidspatienten (n=38) folgende Besonderheiten auf:

1. Der $B_{12}$-Gehalt des Serums war bei den Patienten weit unterhalb der Norm (um 100 pg/ml).
2. Der Folat-Gehalt des Serums lag bei allen Patienten weit oberhalb der Norm (über 20 ng/ml), obwohl zuvor keine Folsäure appliziert worden war.
3. Obwohl Antikörper gegen Hepatitis B -, Herpes simplex-, Epstein-Barr- und Cytomegalieviren vorhanden waren, verlief der Antikörpertest gegen das Virus, das diesen prefinalen Zustand angeblich verursacht hatte, bei allen Aidspatienten negativ.

Die Zusammenhänge zwischen diesen zunächst unverständlichen Befunden konnten bis Anfang 1988 aufgeklärt werden. Der dahinterstehende Pathomechanismus ist die Cell-folate-depletion.
Seit 1988 werden bei HIV-positiven Patienten routinemäßig folgende Laborparameter gemessen:
Folat in Serum, Erythrozyten und mononukleäre Zellen (Lymphozyten und Monozyten)
$B_{12}$ in Serum, Erythrozyten und mononukleäre Zellen
Dabei zeigten sich vor der Therapie folgende Befunde:

a) Folat
im Serum meist im Normbereich
im Erythrozyten im unteren, meist weit unterhalb des Normbereichs
in mononukleären Zellen (außer bei Patienten mit absoluter Lymphozytose) zwischen 30 bis

50% gesunder Vergleichspersonen

b) $B_{12}$
im Serum im unteren, meist unterhalb des Normbereichs
im Erythrozyten immer unterhalb des Serumwertes
in mononukleären Zellen zwischen 20 bis 30% gesunder Vergleichspersonen

Nach der Therapie war der intrazelluläre Folat- und $B_{12}$-Gehalt in Erythrozyten und mononukleären Zellen bei allen Patienten im Normbereich.
Diese Befunde sprechen für eine Störung im Bereich der fakultativen DNA-Methylierung.

zu 7.: Der "Aids-Test" (HIV-Elisa) ist ein quantitativer Test. Bei dem Versuch der Quantifizierung (Titrierung) zeigte sich bei zwei Therapien 1987:
HIV-AB-Titer:

| | | |
|---|---|---|
| vor Therapie 1: | 1 : | 2.621.440 |
| nach 3 Monaten: | 1 : | 10.240 |
| vor Therapie 2: | 1 : | 327.680 |
| nach 3 Monaten: | 1 : | 5.120 |

Da bei diesen beiden Therapien mit einer klinisch sichtbaren (vor allem cerebralen) Verbesserung der Patienten einhergingen, war der Schluß zulässig, daß dem Antikörperabfall (Lebenszeit von Plasmazellen ca. 1 1/2 Monate) eine Reduzierung des Antigens voranging.
Diese Befunde sprechen für eine mögliche Störung im Bereich der fakultativen DNA-Methylierung, besonders aber für eine Störung im Bereich der obligatorischen DNA-Methylierung.

zu 8.: HIV-Westernblot:
Verwendet wurde der DuPont-Test-Kit: gP 160; gP 120; gp41; P 55,; P 24
Grundsätzlich werden hierbei Virusproteine elektrophoretisch getrennt und auf Nitrozellulosestreifen fixiert. Gemessen wird dann die Anwesenheit von Antikörpern gegen diese spezifischen Virusproteine.
Da sich bei HIV nicht um ein Virus im üblichen Sinne handelt, sondern um ein RNA-Intermediat, entstanden durch behinderte Genamplifikationen bei Differenzierungsvorgängen, ist die Beurteilung eines HIV-Westernblots eingeschränkt durch zwei Kriterien:

1. Welche Proteine kommen bei einem beeinträchtigten Differenzierungsvorgang in das Serum?

2. Wie groß ist die Fähigkeit eines Patienten zur Bildung spezifischer Antikörper?

Einen entsprechenden Westernblot sowie eine Übersicht über den HIV-1-Antikörpernachweis im Immunblot zeigen die beigefügten Figuren 2 und 3.
Nach allen Therapien war eine Zunahme der Antikörperspezifität zu verzeichnen.
Diese Befunde sprechen für eine Störung sowohl im Bereich der fakultativen als auch der obligatorischen DNA-Methylierung.

zu 9., 10. und 11.: β-2-Mikroglobulin, Neopterin und Lysozym wurde bei keinem Patienten durch die Therapie signifikant geändert.

zu 12.: Serologische Untersuchungen sind vor allem bei Erkrankungen, die das Immunsystem kompromittieren, nicht zuverlässig.
So entwickelten z.B. fünf Patienten nach Therapie einen positiven Cardiolipin-Test, eine Patientin einen positiven FTA-IgG-Test. Bei einer Patientin konnte mittels PCR (polymerase chain reaction) dreimal Antigen von Treponema pallidum on Borrelia Burgdorferi nachgewiesen werden; dieserologischen Reaktionen blieben aber immer negativ.

Ganz allgemein sollte daher dem direkten Antigennachweis (z.B. PCR) der Vorzug gegeben werden.

Erfindungsgemäß können die Arzneimittel selbst oder eines ihrer pharmazeutisch annehmbaren Salze mit pharmazeutisch annehmbaren Säuren, wie beispielsweise Milchsäure, Zitronensäure, Äpfelsäure, Fumarsäure oder Weinsäure, oder mit pharmazeutisch annehmbaren Basen, wie Alkali- oder Erdalkalihydroxiden, -carbonaten, organischen Aminen oder Ammoniak, verwendet werden.

Die erfindungsgemäßen Arzneimittel oder ihre pharmazeutisch annehmbaren Salze können bei der Human- oder Veterinärmedizin verwendet werden.

Die Dosis, mit der die erfindungsgemäßen Arzneimittel verabreicht werden, wird von dem Verabreichungsweg, dem Körpergewicht des Patienten und dem zu behandelnden Zustand sowie seiner Stärke abhängen.

Eine vorgeschlagene Dosis der Verbindungen der Gruppe Thymin, Thymidin, desoxy-Thymidin, desoxy-Thymidin-mono-, -di- und -triphosphat oder eines ihrer pharmazeutisch annehmbaren Salze beträgt 0,5 bis 1,5 g, ausgedrückt als Gewicht der freien Säuren. Vitamin $B_{12}$ wird bevorzugt in einer Dosierung von 0,1 bis 20 mg pro Tag, mehr bevorzugt 0,5 bis 5 mg pro Tag und besonders bevorzugt 1 bis 2 mg pro Tag verabreicht. Dabei sollte Hydroxy-, Aquo- oder Methylcobalamin verwendet werden. Vitamin $B_6$ wird bevorzugt in einer Dosierung von 10 mg bis 1200 mg pro Tag, mehr bevorzugt 50 bis 600 mg pro Tag, besonders bevorzugt 100 mg pro Tag verabreicht. Methionin wird bevorzugt in einer Dosierung von 0,5 bis 4 g pro Tag, mehr bevorzugt 1 bis 2 g pro Tag und besonders bevorzugt 1,5 g pro Tag verabreicht. Penicillin G wird bevorzugt in einer Dosierung von 5 - 80 Millionen Einheiten pro Tag, mehr bevorzugt 20 bis 40 Millionen Einheiten pro Tag und besonders bevorzugt 30 Millionen Einheiten pro Tag berabreicht. Penicillin G wird bevorzugt parenteral verabreicht, aber auch andere Verabreichungsformen sind denkbar.

Die Verbindung der Gruppe A. (Vitamin $B_6$, Vitamin $B_{12}$, Methionin), der Gruppe B. (Thymin, Thymidin, desoxy-Thymidin, desoxy-Thymidin-mono-, -di- und triphosphat) und der Gruppe C. können zur gleichzeitigen oder getrennten Verabreichung formuliert sein. Es ist bevorzugt, daß die Verbindungen der Gruppe A. und die Verbindungen der Gruppe B. in getrennten Dosiseinheiten vorliegen. Im Falle eines getrennten Vorliegens der Verbindungen der Gruppe A. und der Gruppe B. kann das Penicillin G bzw. die Verbindungen der Gruppe C. in den jeweiligen Monopräparaten enthalten sein. Die Verbindung der Gruppe C. kann auch als getrennte Dosiseinheit vorliegen.

Es ist besonders bevorzugt, daß die Verbindung der Gruppe A. in getrennten Einheit vorliegt, die Verbindung der Gruppe B. zusammen mit der Verbindung der Gruppe A. vorliegt und daß beide Formen jeweils die Verbindung der Gruppe C. enthalten.

Es ist bevorzugt, einen Patienten zuerst mit den Verbindungen der Gruppe Vitamin $B_6$, Vitamin $B_{12}$, Methionin und deren pharmazeutisch annehmbaren Salze bis zum Nachweis von Homocystein im Urin zu behandeln. Üblicherweise dauert diese Therapie etwa vier Wochen. Sie kann aber auch nur zwei Wochen oder sechs Wochen dauern und hängt von der Schwere des zu behandelnden Zustands ab. Während der Therapie mit der Verbindung aus der Gruppe A. kann gleichzeitig die Verbindung aus der Gruppe C. mit verabreicht werden. Wird Homocystein im Urin nachgewiesen, beginnt die Behandlung mit den Verbindungen aus der Gruppe B., d.h. Thymin, Thymidin, desoxy-Thymidin, desoxy-Thymidin-mono-, -di-, -triphosphat und deren pharmazeutisch annehmbaren Salze. Diese Behandlung dauert im allgemeinen einige Wochen bis einige Monate. Je nach Art der Erkrankung ist es zweckmäßig, gleichzeitig zu den Verbindungen aus der Gruppe A. und B. die Verbindung aus der Gruppe C. zu verabreichen. Die Verabreichung der Verbindung aus der Gruppe C. kann gleichzeitig, getrennt oder zeitlich abgestuft erfolgen.

Die erfindungsgemäßen Arzneimittel bzw. die erfindungsgemäß verwendeten Arzneimittel können in an sich bekannter Weise unter Verwendung von einem oder mehreren physiologisch annehmbaren Trägern oder Arzneimittelverdünnungsmitteln formuliert werden. Präparate für die Verabreichung auf oralem Weg sind bevorzugt. Beispielsweise können Tabletten wie oben ausgeführt die Verbindungen aus den Gruppen A., B. und C. getrennt enthalten oder sie können beliebige Kombinationen aus den Verbindungen der Gruppen A., B. und C. enthalten. Bevorzugt liegt das Arzneimittel in Einheitsdosisform vor.

Präparate für die orale Verwendung, wie Tabletten und Kapseln, können nach an sich bekannten Verfahren mit pharmazeutisch annehmbaren Verdünnungsmitteln, wie Bindemitteln (beispielsweise vorgelatinisierte Maisstärke, Polyvinylpyrrolidon oder Hydroxypropylmethylcellulose); Füllstoffen (beispielsweise Lactose, mikrokristalline Cellulose oder Calciumhydrogenphosphat); Schmiermitteln (beispielsweise Magnesiumstearat, Talk oder Siliciumdioxid); Desintegrationsmitteln (beispielsweise Kartoffelstärke oder Natriumstärkeglykolat); oder Benetzungsmitteln (beispielsweise Natriumlaurylsulfat), hergestellt werden. Tabletten können nach an sich gut bekannten Verfahren beschichtet werden.

Flüssige Präparationen für die orale Verabreichung können in Form von beispielsweise Lösungen, Sirupen oder Suspensionen vorliegen, oder sie können als Trockenprodukt für die Rekonstitution mit Wasser oder einem geeigneten anderen Träger vor der Verwendung vorliegen. Solche flüssigen Präparationen können nach an sich bekannten Verfahren mit pharmazeutisch annehmbaren Zusatzstoffen, wie Suspensionsmitteln (Sorbitsirup, Cellulosederivate oder hydrierte genießbare Fette); Emulgiermitteln (beispielsweise Lecithin oder Acacia); nichtwäßrigen Trägern (beispielsweise Mandelöl, ölige Ester, Ethylalkohol oder fraktionierte Pflanzenöle); und Konservierungsstoffen (beispielsweise Methyl- oder Propyl-p-hydroxy-benzoaten oder Sorbinsäure), hergestellt werden. Die Präparationen können ebenfalls Puffersalze, Geschmacksmittel, Farbstoffe und Süßmittel, je nach Bedarf, enthalten.

Zubereitungen für die orale Verabreichung können geeigneterweise so formuliert sein, daß eine kontrollierte Freigabe der aktiven Bestandteile erfolgt.

Für die parenterale Verabreichung können die Zubereitungen in Form einer für die Bolus-Injektion oder kontinuierliche Infusion geeigneten Form vorliegen. Die Zubereitungen für die Injektion können in Dosiseinheitsform, beispielsweise in Ampullen, oder in Behältern mit mehreren Dosiseinheiten mit einem zugegebenen Konservierungsstoff vorliegen. Die Präparate können in Form von Suspensionen, Lösungen oder Emulsionen in öligen oder Wäßrigen Trägern vorliegen, und sie können Formulierungshilfsstoffe, wie Suspensions-, Stabilisierungs- und/oder Dispersionsmittel, enthalten. Alternativ können die aktiven Bestandteile in Pulverform für die Rekonstitution mit einem geeigneten Träger, beispielsweise sterilem pyrogenfreien Wasser, vor der Verwendung vorliegen.

Für die rektale Verabreichung können die Zubereitungen als Suppositorien oder als Retentions-Klistiere vorliegen, beispielsweise als solche, welche an sich bekannte Suppositorien-Grundstoffe, wie Kakaobutter oder andere Glyceride, enthalten.

Die erfindungsgemäßen bzw. erfindungsgemäß verwendeten pharmazeutischen Arzneimittel können nach an sich bekannten Verfahren, die in der pharmazeutischen Industrie gut bekannt sind, hergestellt werden.

Beispielsweise können die erfindungsgemäßen bzw. erfindungsgemäß verwendeten Arzneimittel oder eines ihrer Salze gegebenenfalls mit geeigneten Verdünnungsmitteln vermischt werden. Die Tabletten können beispielsweise durch direkte Verpressung eines solchen Gemisches hergestellt werden. Kapseln können hergestellt werden, indem das Gemisch zusammen mit geeigneten Verdünnungsmitteln in Gelatinekapseln unter Verwendung einer geeigneten Füllmaschine eingefüllt wird. Formulierungen mit kontrollierter Freigabe für die orale oder rektale Verabreichung können in an sich bekannter Weise zubereitet werden, wie es für Formen mit kontrollierter Freigabe bzw. verzögerter Freigabe bekannt ist.

Die erfindungsgemäßen bzw. erfindungsgemäß verwendeten Arzneimittel können gegebenenfalls in einer Mehrfachpackung oder einer Abgabevorrichtung vorliegen, welche eine oder mehrere Dosiseinheitsformen, die die aktiven Bestandteile enthalten, enthält. Die Packungen können beispielsweise Metall- oder Kunststoffolie enthalten, wie eine Blisterpackung bzw. Durchsteckpackung. Die Packung oder die Abgabevorrichtung kann Bedienungsanleitungen bzw. Anleitungen für die Verabreichung enthalten. Wenn die Verbindungen der Gruppe A. und der Gruppe B. und gegebenenfalls die Verbindung der Gruppe C. oder jeweils eines ihrer pharmazeutisch annehmbaren Salze als drei getrennten Präparate verabreicht werden, können diese beispielsweise in Form einer Mehrfachpackung vorliegen.

Die Erfindung wird an Hand der folgenden Beispiele näher erläutert.

Die folgenden Beispiele erläutern erfindungsgemäße Arzneimittel näher. Andere erfindungsgemäße Formulierungen können auf ähnliche Weise zubereitet werden.

## Beispiel A

Tabletten

Tabletten können nach den üblichen Verfahren, wie durch direktes Verpressen oder durch Naßgranulierung hergestellt, werden. Die Tabletten können mit geeigneten filmbildenden Materialien, wie Hydroxypropylmethylcellulose, unter Verwendung von Standardverfahren filmbeschichtet werden.

| (i) Direkte Verpressung | |
|---|---|
| | mg/Tablette |
| aktiver Bestandteil (Verbindung aus der Gruppe B.) | 1140 mg |
| Lactose | 435 mg |
| mikrokristalline Cellulose | 420 mg |
| vernetztes Polyvinylpyrrolldon | 84 mg |
| Magnesiumstearat | 21 mg |
| Kompressionsgewicht | 2100 mg |

Der aktive Bestandteil, die mikrokristalline Cellulose, die Lactose und das vernetzte Polyvinylpyrrolidon werden

durch ein 500-µm-Sieb gesiebt und in einem geeigneten Mischer vermischt. Das Magnesiumstearat wird durch ein 250-µm-Sieb gesiebt und mit dem aktiven Bestandteil vermischt. Das Gemisch wird zu Tabletten unter Verwendung geeigneter Stanzlöcher verpreßt.

| (ii) Naßgranulierung | |
|---|---|
| | mg/Tablette |
| aktiver Bestandteil (Verbindung aus der Gruppe B.) | 1140 mg |
| Lactose | 645 mg |
| vorgelatinisierte Stärke | 210 mg |
| vernetztes Polyvinylpyrrolidon | 84 mg |
| Magnesiumstearat | 21 mg |
| Kompressionsgewicht | 2100 mg |

Der aktive Bestandteil, die Lactose und die vorgelatinisierte Stärke werden zusammen vermischt und mit Wasser granuliert. Die nasse Masse wird getrocknet und vermahlen. Das Magnesiumstearat und das vernetzte Polyvinylpyrrolidon werden durch ein 250-µm-Sieb gesiebt und mit dem Granulat vermischt. Das entstehende Gemisch wird unter Verwendung geeigneter Tabletten-Lochformen verpreßt.

**Beispiel B**

Lutschbare bzw. kaubare Tabletten

| | | mg/Tablette |
|---|---|---|
| (i) | aktiver Bestandteil (Verbindung aus der Gruppe B) | 1140 mg |
| | Polyvinylpyrrolidon | 84 mg |
| | Süßstoff/Geschmacksstoff | q.s. |
| | Magnesiumstearat | 21 mg |
| | Mannit bis zu | 2100 mg |
| | Kompressionsgewicht | 2100 mg |

Der aktive Bestandteil, der Süßstoff/Geschmacksstoff und das Mannit werden zusammen vermischt und mit einer Lösung aus Polyvinylpyrrolidon granuliert. Die nasse Masse wird getrocknet, vermahlen und mit Magnesiumstearat (welches durch ein 250-µm-Sieb gesiebt wurde) als Schmiermittel versetzt.

Das entstehende Granulat wird zu Tabletten unter Verwendung geeigneter Lochformen verpreßt.

| | | mg/Tablette |
|---|---|---|
| (ii) | aktiver Bestandteil (Verbindung aus der Gruppe B.) | 1140 mg |
| | Hydroxypropylmethylcellulose | 60 mg |
| | Magnesiumstearat | 21 mg |
| | Geschmacksstoff | q.s. |
| | Xylit bis zu | 2100 mg |
| | Kompressionsgewicht | 2100 mg |

Der aktive Bestandteil, das Xylit und die Geschmacksstoffe werden zusammen vermischt, unter Verwendung einer Lösung von Hydroxypropylmethylcellulose in wäßrigem Ethanol granuliert und getrocknet. Das Granulat wird gemahlen, mit Magnesiumstearat (welches durch ein 250-µm-Sieb gesiebt wurde) als Schmiermittel versetzt und zu Tabletten unter Verwendung geeigneter Lochformen verpreßt.

**Beispiel C**

Kapseln

| | | mg/Kapsel |
|---|---|---|
| (i) | aktiver Bestandteil (Verbindung aus der Gruppe B.) | 1140 mg |
| | vorgelatinisierte Stärke | 195 mg |
| | Magnesiumstearat | 15 mg |
| | Füllgewicht | 1350 mg |

Der aktive Bestandteil und die vorgelatinisierte Stärke werden durch ein 500-µm-Sieb gesiebt, zusammen vermischt und mit Magnesiumstearat (welches durch ein 250-µm-Sieb geseibt wurde) als Schmiermittel versetzt. Das Gemisch wird in Hartgelatinekapseln geeigneter Größe eingefüllt.

| | | mg/Kapsel |
|---|---|---|
| (ii) | aktiver Bestandteil (Verbindung aus der Gruppe B.) | 1140 mg |
| | Lactose | 225 mg |
| | Polyvinylpyrrolidon | 60 mg |
| | vernetztes Polyvinylpyrrolidon | 60 mg |
| | Magnesiumstearat | 15 mg |
| | Füllgewicht | 1500 mg |

Der aktive Bestandteil und die Lactose werden zusammen vermischt und mit einer Lösung aus Polyvinylpyrrolidon naß vermischt. Die Masse wird getrocknet und gemahlen und mit vernetztem Polyvinylpyrrolidon und Magnesiumstearat (gesiebt durch ein 250-µm-Sieb) vermischt. Das entstehende Gemisch wird in Hartgelatinekapseln

geeigneter Größe abgefüllt.

**Beispiel D**

Sirup für die orale Verwendung

| | |
|---|---|
| aktiver Bestandteil (Verbindung aus der Gruppe B.) | 1140 mg |
| Hydroxypropylmethylcellulose | 135 mg |
| Propylhydroxybenzoat | 45 mg |
| Butylhydroxybenzoat | 2,25 mg |
| Natriumsaccharin | 15 mg |
| Sorbitlösung | 3 ml |
| geeigneter Puffer | q.w. |
| geeignete Geschmacksstoffe | q.s. |
| gereinigtes Wasser bis zu | 3 ml |

Die Hydroxypropylmethylcellulose wird in einem Teil des heißen gereinigten Wassers zusammen mit den Hydroxybenzoaten dispergiert, und die Lösung wird auf Raumtemperatur abkühlen gelassen. Das Natriumsaccharin, die Geschmacksstoffe und die Sorbitlösung werden zu der Massen-Lösung zugegeben. Der aktive Bestandteil wird in einem Teil des verbleibenden Wassers gelöst und zu der Masse der Lösung gegeben. Geeignete Puffer können zur Kontrolle des pH-Wertes im Bereich einer maximalen Stabilität zugegeben werden. Die Lösung wird auf das Volumen aufgefüllt, filtriert und in geeignete Behälter gefüllt.

**Patentansprüche**

**1.** Verwendung von mindestens einer Verbindung aus der Gruppe

A. Vitamin $B_6$, Vitamin $B_{12}$, Methionin und eines pharmazeutisch annehmbaren Salzes davon
zusammen mit mindestens einer Verbindung aus der Gruppe

B. Thymin, Thymidin, desoxy-Thymidin, desoxy-Thymidin-mono-, -di- und -triphosohat und eines pharmazeutisch annehmbaren Salzes davon
und gegebenenfalls

C. mit Penicillin G und/oder einem pharmazeutisch annehmbaren Salz davon
zur Herstellung eines Arzneimittels zur Behandlung und/oder Prophylaxe von Störungen des Folat-Stoffwechsels, wobei die Verwendung der Verbindungen aus der Gruppe A. und B. gleichzeitig, getrennt oder zeitlich abgestuft erfolgen kann und die Verwendung von Penicillin G zusammen mit der Verwendung der Verbindungen aus der Gruppe A. und/oder aus der Gruppe B. erfolgen kann.

**2.** Verwendung nach Anspruch 1 zur Behandlung und/oder Prophylaxe von AIDS und Aids-Related-Complex (ARC), Syphilis, Chronic-Immuno-Deficiency-Syndrome (CIDS) bzw. Chronic-Fatigue-Syndrome (CFS), multipler Sklerose (MS), Neoplasien und Malaria, wobei die Verwendung bei Neoplasien und Malaria ohne Penicillin G erfolgt.

**3.** Verwendung nach Anspruch 1 oder 2, dadurch **gekennzeichnet,** daß das Arzneimittel in Dosiseinheitsform vorliegt, die 10 mg bis 1200 mg Vitamin $B_6$, 0,1 bis 20 mg Vitamin $B_{12}$, 0,5 bis 4 g Methionin, 0,5 bis 1,5 g Thymin, Thymidin, desoxy-Thymidin, desoxy-Thymidin-mono-, -di- und -triphosphat und 5 bis 80 Millionen Einheiten Penicillin G pro Dosiseinheit oder jeweils ein pharmazeutisch annehmbares Salz davon enthält.

4. Verwendung nach mindestens einem der Ansprüche 1 bis 3, dadurch **gekennzeichnet,** daß die Verbindungen der Gruppe A. und B. in oral verabreichbarer Form und die Verbindung der Gruppe C. in parenteral verabreichbarer Form vorliegen.

5. Mehrfachpackung, dadurch **gekennzeichnet,** daß sie getrennte Dosiseinheiten aus mindestens einer Verbindung der Gruppe

A. Vitamin $B_6$, Vitamin $B_{12}$, Methionin und eines pharmazeutisch annehmaren Salzes davon
zusammen mit mindestens einer Verbindung aus der Gruppe

B. Thymin, Thymidin, desoxy-Thymidin, desoxy-Thymidin-mono-, -di- und -triphosphat und eines pharmazeutisch annehmbaren Salzes davon
sowie gegebenenfalls

C. Penicillin G oder ein pharmazeutisch annehmbares Salz davon
jeweils zusammen mit üblichen Trägern und Verdünnungsmitteln, enthält.

**Claims**

1. Use of at least one compound selected from the group:

A. vitamin $B_6$, vitamin $B_{12}$, methionine and a pharmaceutically acceptable salt thereof
together with at least one compound selected from the group:

B. thymine, thymidine, deoxythymidine, deoxythymidine monophosphate, deoxythymidine diphosphate, deoxythymidine triphosphate and a pharmaceutically acceptable salt thereof
and optionally

C. with penicillin G and/or a pharmaceutically acceptable salt thereof
for the preparation of a medicament for the treatment and/or prophylaxis of disorders of folate metabolism, wherein the compounds selected from Groups A and B may be used simultaneously, separately or in temporally staggered manner and penicillin G may be used together with the compound or compounds selected from Group A and/or from Group B.

2. Use according to Claim 1 for the treatment and/or prophylaxis of AIDS and AIDS-Related Complex (ARC), syphilis, Chronic Immuno-Deficiency Syndrome (CIDS) or Chronic-Fatigue Syndrome (CFS), multiple sclerosis (MS), neoplasias and malaria, wherein use is effected without penicillin G in the case of neoplasias and malaria.

3. Use according to Claim 1 or 2, characterised in that the medicament is present in dose-unit form that contains 10 mg to 1,200 mg vitamin $B_6$, 0.1 to 20 mg vitamin $B_{12}$, 0.5 to 4 g methionine, 0.5 to 1.5 g thymine, thymidine, deoxythymidine, deoxythymidine monophosphate, deoxythymidine diphosphate and deoxythymidine triphosphate and 5 to 80 million units of penicillin G per dose unit or, in each instance, a pharmaceutically acceptable salt thereof.

4. Use according to at least one of Claims 1 to 3, characterised in that the compounds pertaining to Groups A and B are present in orally dispensable form and the compound pertaining to Group C is present in parenterally dispensable form.

5. Multiple packaging, characterised in that it contains separate dose units consisting of at least one compound selected from the group:

A. vitamin $B_6$, vitamin $B_{12}$, methionine and a pharmaceutically acceptable salt thereof
together with at least one compound selected from the group:

B. thymine, thymidine, deoxythymidine, deoxythymidine monophosphate, deoxythymidine diphosphate, deoxythymidine triphosphate and a pharmaceutically acceptable salt thereof
and optionally

C. penicillin G and/or a pharmaceutically acceptable salt thereof

in each instance together with conventional excipients and diluents.

**Revendications**

1. Utilisation d'au moins un composé choisi dans le groupe

   A. vitamine $B_6$, vitamine $B_{12}$, méthionine et un sel pharmaceutiquement acceptable de celles-ci
   conjointement avec au moins un composé choisi dans le groupe
   B. thymine, thymidine, désoxy-thymidine, mono-, di- et triphosphate de désoxy-thymidine et un sel pharmaceutiquement acceptables de ceux-ci
   et éventuellement
   C. avec de la pénicilline G et/ou un sel pharmaceutiquement acceptable de celle-ci
   pour la préparation d'un médicament pour le traitement et/ou la prophylaxie des troubles du métabolisme du folate, l'utilisation des composés des groupes A et B pouvant se faire simultanément, séparément ou de façon échelonnée dans le temps, et l'utilisation de la pénicilline G pouvant se faire conjointement avec l'utilisation des composés du groupe A et/ou du groupe B.

2. Utilisation selon la revendication 1 pour le traitement et/ou la prophylaxie du SIDA et du syndrome associé au SIDA (para-SIDA), de la syphilis, du syndrome d'immunodéficience chronique (SIDC), du syndrome de fatigue chronique (SFC), de la sclérose en plaques (SEP), des néoplasies et de la malaria, l'utilisation se faisant sans la pénicilline G dans le cas des néoplasies et de la malaria.

3. Utilisation selon la revendication 1 ou 2, caractérisée en ce que le médicament se présente sous la forme de doses unitaires qui contiennent 10 mg à 1200 mg de vitamine $B_6$, 0,1 à 20 mg de vitamine $B_{12}$, 0,5 à 4 g de méthionine, 0,5 à 1,5 g de thymine, de thymidine, de désoxy-thymidine, de mono-, di- ou triphosphate de désoxy-thymidine et 5 à 80 millions d'unités de pénicilline G par dose unitaire ou à chaque fois d'un sel pharmaceutiquement acceptable de ceux-ci.

4. Utilisation selon au moins l'une des revendications 1 à 3, caractérisée en ce que les composés des groupes A et B se présentent sous une forme pouvant être administrée par voie orale, et le composé du groupe C se présente sous une forme pouvant être administrée par voie parentérale.

5. Emballage multiple caractérisé en ce qu'il contient des doses unitaires séparées d'au moins un composé choisi dans le groupe

   A. vitamine $B_6$, vitamine $B_{12}$, méthionine et un sel pharmaceutiquement acceptable de celles-ci
   conjointement avec au moins un composé choisi dans le groupe
   B. thymine, thymidine, désoxy-thymidine, mono-, di- et triphosphate de désoxy-thymidine et un sel pharmaceutiquement acceptables de ceux-ci
   ainsi qu'éventuellement
   C. de la pénicilline G ou un sel pharmaceutiquement acceptable de celle-ci
   à chaque fois conjointement avec des véhicules et des diluants usuels.

Figur 1

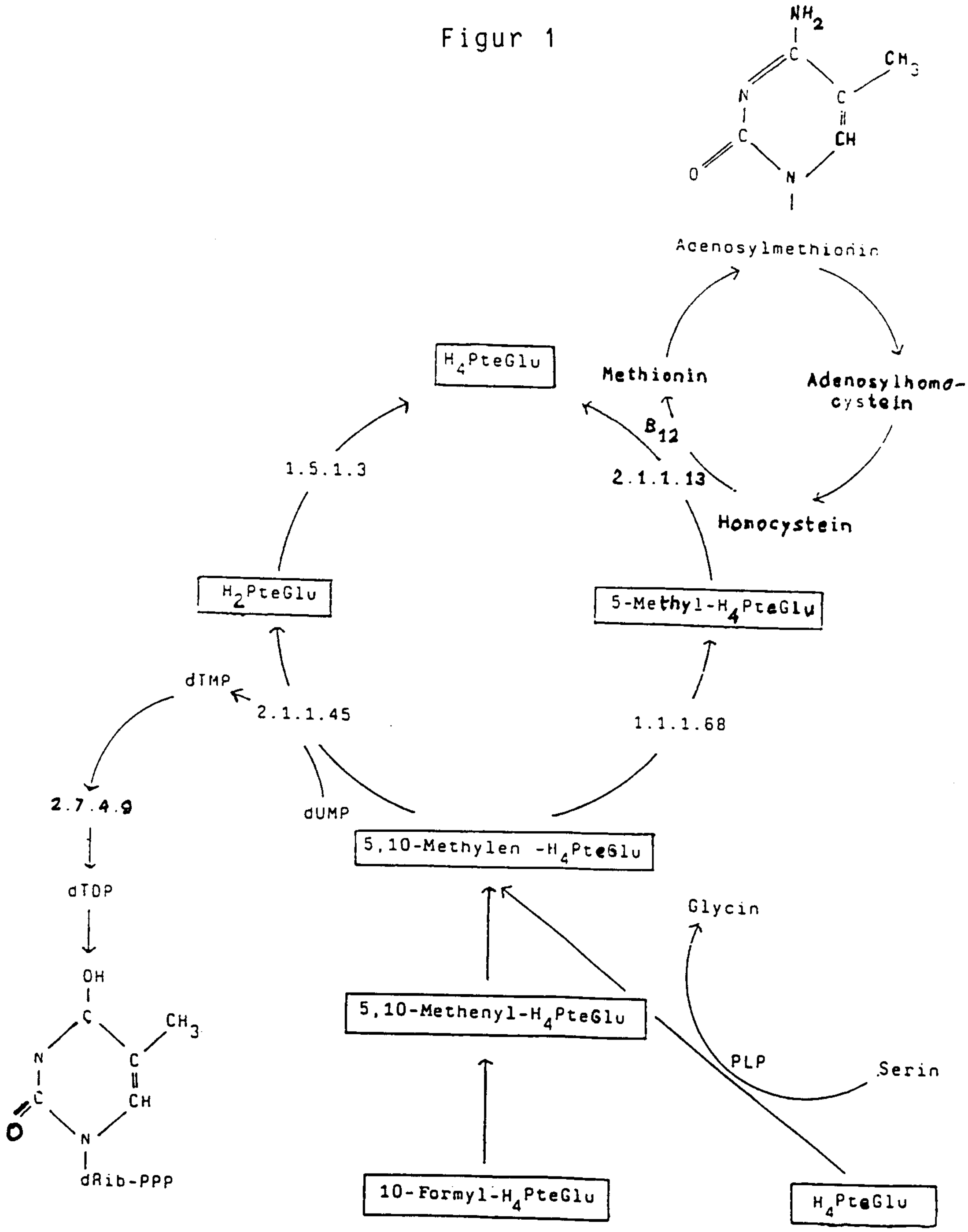
Adenosylmethionin
Methionin
Adenosylhomo-cystein
$B_{12}$
2.1.1.13
Homocystein
$H_4$PteGlu
1.5.1.3
$H_2$PteGlu
dTMP
2.1.1.45
2.7.4.9
dTDP
dUMP
5-Methyl-$H_4$PteGlu
1.1.1.68
5,10-Methylen -$H_4$PteGlu
Glycin
5,10-Methenyl-$H_4$PteGlu
PLP
Serin
10-Formyl-$H_4$PteGlu
$H_4$PteGlu

Figur 2

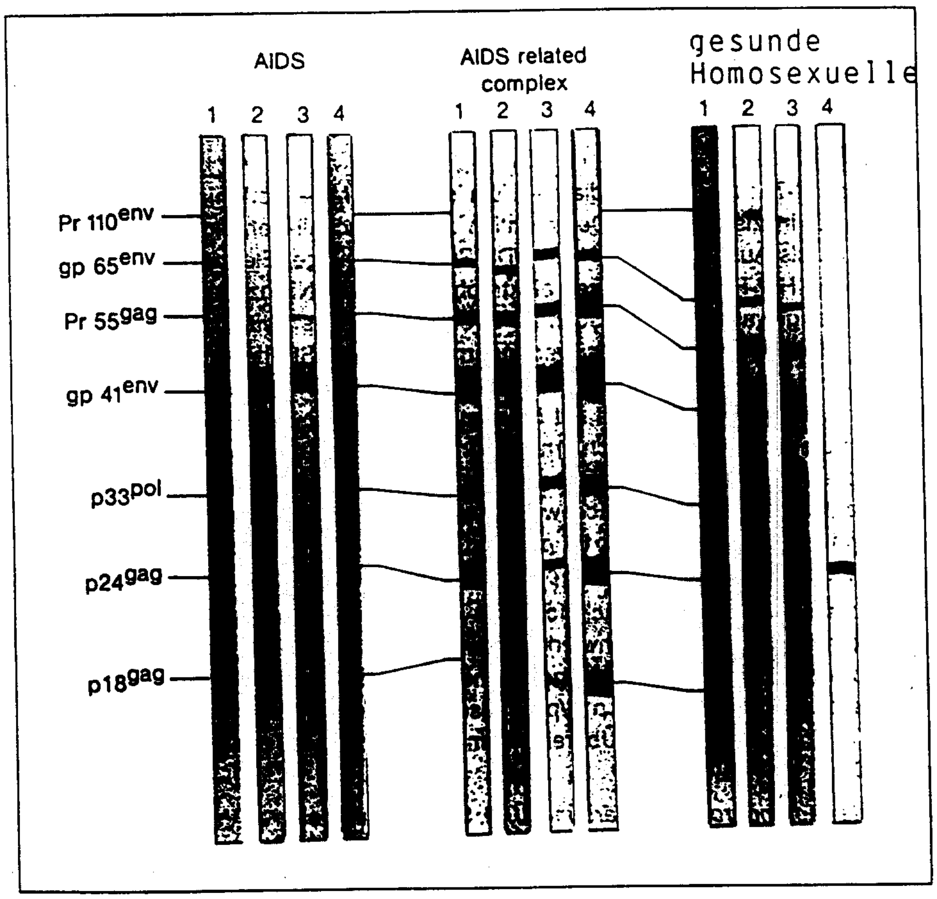
AIDS
AIDS related complex
gesunde Homosexuelle
1 2 3 4
1 2 3 4
1 2 3 4
Pr 110env
gp 65env
Pr 55gag
gp 41env
p33pol
p24gag
p18gag

Figur 3

| Virusproteine | | | Befund* | |
|---|---|---|---|---|
| Molekülmasse (Dalton) | Abk. | Typ | Aussehen der Immunoblot-Farbbanden | Vorkommen bei HIV-1-Infektion |
| 160.000 | gp160 | envelope (Vorläufer des gp120) | breite, diffuse Bande | häufig in Verbindung mit gp120 |
| 120.000 | gp120 | envelope | breite, diffuse Bande | häufig |
| 65.000 | p65 | Polymerase | schmale Bande | rel. häufig |
| 53.000 – 55.000 | p53 – 55 | Protein-precursor (gag) | schmale Bande | häufig in Verbindung mit p24 |
| 41.000 – 43.000 | gp41 – 43 | envelope (Transmembran-Protein) | breite Bande, verwaschene Randzonen | sehr häufig, env-Hauptbande |
| 30.000 | p30 | Endonuclease | schmale Bande | rel. häufig |
| 24.000 | p24 | core | breite Bande | sehr häufig (AIDS-Vorstadien), core-Hauptbande |
| 18.000 | p18 | Internes Protein (gag) | schmale Bande | relativ häufig |

* gp41-Nachweis u. ggf. weitere spezifische Banden = HIV-1 positiv, ebenso p24 neben gp41 und/oder gp120, 160. p24-Nachweis allein oder weitere Banden (außer gp41, gp120) = HIV-1 fraglich positiv, spätere Befundkontrolle erforderlich.